# EUROPÄISCHE PATENTSCHRIFT

(11) **EP 1 745 134 B1**
(45) Veröffentlichungstag und Bekanntmachung des Hinweises auf die Patenterteilung: **25.02.2009**
(21) Anmeldenummer: 05743972.1
(22) Anmeldetag: 29.04.2005
(51) Int. Cl.: C12P 7/16

(54) **VERFAHREN ZUR HERSTELLUNG VON 2-BUTANOL DURCH ENZYMATISCHE REDUKTION VON 2-BUTANON IN EINEM ZWEI-PHASEN-SYSTEM**
PROCESS FOR THE PRODUCTION OF 2-BUTANOL BY ENZYMATIC REDUCTION OF 2-BUTANONE IN A TWO PHASE SYSTEM
PROCÉDÉ DE PRODUCTION DE 2-BUTANOL PAR RÉDUCTION ENZYMATIQUE DE 2-BUTANONE DANS UN SYSTÈME BIPHASIQUE

(30) Priorität: 10.05.2004 AT 8002004
(43) Veröffentlichungstag der Anmeldung: 24.01.2007
(73) Patentinhaber: IEP GmbH, 65203 Wiesbaden (DE)
(72) Erfinder: GUPTA, Antje, 65207 Wiesbaden (DE); Tschentscher, Anke, 65347 Eltville-Hattenheim (DE); BOBKOVA, Maria, 65510 Idstein (DE)
(74) Vertreter: Schwarz, Albin
(86) Internationale Anmeldenummer: PCT/IB2005/001556
(87) Internationale Veröffentlichungsnummer: WO 2005/108593

(56) Entgegenhaltungen:
- EP-A- 1 323 827
- WO-A-93/18138
- WO-A-20/04111083
- WO-A-20/05049816

## Beschreibung

Die Erfindung betrifft ein Verfahren zur Herstellung von 2-Butanol und insbesondere R-2-Butanol und S-2-Butanol durch enzymatisch-katalysierte Reduktion von 2-Butanon mit einer Carbonylreduktase und einem Co-Enzym.

2-Butanol und insbesondere die chiralen Verbindungen R-2-Butanol und S-2-Butanol sind begehrte Zwischenprodukte bei der Herstellung pharmazeutisch wirksamer Substanzen.

Die Herstellung von enantiomerenreinem 2-Butanol, also R-2-Butanol und S-2-Butanol, ist aufwendig, da eine direkte chemische, katalytische asymmetrische Reduktion von 2-Butanon zu R- bzw. S-2-Butanol bis heute nicht möglich ist. Auch Verfahren zur direkten enzymatischen Reduktion sind bisher nicht beschrieben. Enantiomerenreines R-2-Butanol und S-2-Butanol sind im großtechnischen Maßstab nur auf Umwegen über Razematspaltung herzustellen.

Carbonylreduktasen (weitere Bezeichnungen: Alkoholdehydrogenasen, Oxidoreduktasen) sind als Katalysatoren zur Reduktion von Carbonylverbindungen bzw. zur Oxidation von sekundären Alkoholen bekannt. Diese Enzyme benötigen ein Co-Enzym, z.B. NAD(P)H. Die Reduktion von Ketonen mit der aus Lactobacillus kefir gewonnenen Carbonylreduktase und dem Co-Enzym NADPH ist z.B. aus der US 5,342,767 bekannt.

Die Reinigung und Charakterisierung einer Alkoholdehydrogenase aus Moraxella sp. ist aus Eur. J. Biochem. 254, 356-362 (1998) bekannt.

Die Reduktion von 2-Butanon zu 2-Butanol mittels einer Carbonylreduktase in einem wässerigen Medium ist schwierig, da das Reaktionsgemisch nicht leicht aufzuarbeiten ist und 2-Butanol in Wasser sehr gut löslich ist. Außerdem gestalten sich extraktive und destillative Verfahren zur Trennung von 2-Butanol und Wasser technisch aufwendig.

Ein weiteres Problem bei der enzymatischen Reduktion von 2-Butanon zu 2-Butanol mittels einer Carbonylreduktase stellt die Regenerierung des Cofaktors NADH bzw. NADPH dar. Die heute oft angewendete Methode der Regeneration des NAD(P)H mit 2-Propanol ist hier ebenfalls problematisch, da dieses die Isolierung des Produktes R- bzw. S-2-Butanol zusätzlich erschwert und ebenfalls nur äußerst aufwendig vollständig vom R- bzw. S-2-Butanol abtrennbar ist.

Ein zusätzliches Problem bei der enzymatischen Reduktion von 2-Butanon im wäßrigen Medium bei gleichzeitiger Coenzymregenerierung des NAD(P)H mit 2-Propanol stellt die Inaktivierung der meisten Enzyme bei einem Gehalt von 2-Propanol- und 2-Butanol von über 20 % dar. Das bedeutet, daß die letzliche Konzentration an einzusetzendem 2-Butanon weit unter 10 % (w/v) liegen muss, wenn man von einem Überschuss an einzusetzendem 2-Propanol ausgeht. Diese geringen realisierbaren Substrat bzw. Produktkonzentrationen erschweren wiederum die Isolierung des R- bzw. S-2-Butanol aus dem Reaktionsgemisch.

Das Dokument WO 93/18138 A ((FORSCHUNGSZENTRUM JUELICH GMBH), 16. September 1993) offenbart ein Verfahren zur Herstellung von 2-Butanol durch enzymatisch katalysierte Reduktion von 2-Butanon mit einer Carbonylreduktase aus *Candida parapsilosis* und einem Coenzym in einem einphasigen, wäßrigen System. Die Regenerierung des Coenzyms erfolgt durch die Kopplung mit Natriumformiat/Formiat-Dehydrogenase. Das Dokument EP-A-1 323 827 ((SUMITOMO CHEMICAL COMPANY, LIMITED), 2. Juli 2003) offenbart ein Verfahren zur Herstellung von 2-Hydroxycyclo-alkancarboxylsäureester durch enzymatisch katalysierte Reduktion von 2-Oxocyclo-alkancarboxylsäureester mit einer Carbonylreduktase und einem Coenzym in einem Zwei-Phasen-System und in Gegenwart eines sekundären Alkohols mit einem Siedpunkt von nicht mehr als 200°C.

Das erfindungsgemäße Verfahren zur Herstellung von 2-Butanol durch enzymatisch-katalysierte Reduktion von 2-Butanon mit einer Carbonylreduktase und einem Co-Enzym stellt sich nun die Aufgabe, die oben genannten Probleme zu lösen und ist dadurch gekennzeichnet, daß
(a) eine wässerige Phase, welche die Carbonylreduktase und das Co-Enzym enthält, mit einer alkoholischen Phase, die mit der wässerigen Phase nicht mischbar ist und 2-Butanon enthält, zur Reduktion des 2-Butanons in Kontakt gebracht wird, mit der Maßgabe, daß der in der alkoholischen Phase vorhandene Alkohol ein sekundärer Alkohol ist, der in der Lage ist, das Co-Enzym zu regenerieren und einen Siedepunkt besitzt, der über dem von Wasser liegt, worauf
(b) das gebildete 2-Butanol abgetrennt wird.

Im erfindungsgemäßen Verfahren wird die Umsetzung von 2-Butanon zu 2-Butanol mittels Carbonylreduktase somit in einem Zweiphasensystem durchgeführt, das aus einer wäßrigen Phase, in der das Enzym und der Co-Enzym gelöst sind, und einer organischen Phase, die aus dem sekundären Alkohol und 2-Butanon gebildet ist, besteht.

Die Co-Enzym-Regenerierung mit einem sekundären Alkohol vorgenommen, der nicht mit Wasser mischbar ist und gleichzeitig einen möglichst deutlich höheren Siedepunkt als Wasser aufweist. Als vorteilhaft haben sich dabei 2-Pentanol, 2-Hexanol, 2-Heptanol, 2-Oktanol und 4-Methyl-2-pentanol erwiesen, von denen 2-Heptanol und 2-Oktanol bevorzugt sind.

Als Co-Enzym eignet sich NADH und NADPH ganz besonders. Zusätzlich führt der sekundäre, nicht wassermischbare Alkohol zu einer Stabilisierung der Carbonylreduktase im erfindungsgemäßen Verfahren.

Der Vorteil der Verwendung eines nicht wassermischbaren sekundären Alkohols zur Co-Enzymregenerierung liegt auch darin, daß dieser in höherem Überschuss in Bezug auf das zu reduzierende Substrat 2-Butanon eingesetzt werden kann. Dadurch kann ein höherer Umsatz bei zugleich höheren Konzentrationen an 2-Butanon im Ansatz erreicht werden. Bevorzugt wird der sekundäre Alkohol der alkoholischen Phase und das 2-Butanon daher in einem molaren Verhältnis im Bereich zwischen 1:2 bis 1:10 (2-Butanon : sekundärer Alkohol) eingesetzt, wobei ein molares Verhältnis im Bereich zwischen 1:2,5 bis 1:5 besonders bevorzugt ist.

Eine weitere Ausgestaltung des erfindungsgemäßen Verfahrens besteht darin, dass das 2-Butanon in einer Menge eingesetzt wird, die mindestens 5 Vol.-%, bevorzugt im Bereich von 10 - 25 Vol.-%, bezogen auf das gesamte Reaktionsgemisch, ist.

Die Carbonylreduktase wird bevorzugt in einer Menge von mindestens 2.000 Einheiten, bevorzugt aber mindestens 10.000 Einheiten Carbonylreduktase pro kg 2-Butanon eingesetzt werden, wobei die Obergrenze zweckmäßigerweise 250.000 Einheiten Carbonylreduktase pro kg 2-Butanon beträgt. Der Enzymeinheit 1 U entspricht dabei jener Enzymmenge, die benötigt wird, um 1 µmol 2-Butanon je Minute (min) umzusetzen.

Als Carbonylreduktase bzw. Alkoholdehydrogenase hat sich jene besonders bewährt, welche von Candida parapsilosis stammt. Bevorzugt wird eine Carbonylreduktase eingesetzt, welche die Herstellung von im wesentlichen enantiomer reinem S-2-Butanol oder R-2-Butanol gestattet. Es wurde dabei gefunden, daß die Carbonylreduktase aus Candida parapsilosis in der Lage ist 2-Butanon stereoselektiv zu S-2-Butanol reduzieren, wobei in Abhängigkeit von den gewählten Verfahrensbedingungen ein Enantiomerenüberschuss über 98 % am gewünschten Enantiomer erreichbar ist.

Das im erfindungsgemäßen Verfahren gebildete 2-Butanol befindet sich in der Phase des sekundären Alkohols und kann mit diesem zusammen von der wässerigen Phase dekantiert werden. Anschließend kann das 2-Butanol auf einfache Weise destillativ gewonnen werden.

Im erfindungsgemäßen Verfahren könnte auch der entsprechende enantiomerenreine S-Alkohol zur Koenzymregenerierung eingesetzt werden.

Der Anteil von wäßriger und organischer Phase am Gesamtvolumen des Reaklionsgemisches kann variabel gestaltet werden, wobei der Anteil der wäßrigen Phase bis auf 3 Vol.-% reduziert werden kann, was dazu führt daß sich mehr als 90 % des eingesetzten 2-Butanon bzw. R. bzw. S-2-Butanol in der vom nicht-wassermischbaren Alkohol gebildeten Phase befinden.

Ein weiterer Vorteil des erfindungsgemäßen Verfahrens liegt in der vergleichsweise einfachen Aufarbeitung und Isolierung des R- bzw. S-2-Butanol in hochreiner Form. Die Isolierung des Produktes R- bzw. S-2-Butanol erfolgt durch Abtrennung der organischen Phase und Destillation des 2-Butanon/ 2-Butanol aus dem hochsiedenden nichtwassermischbarem sekundären Alkohol.

Anschließend kann mittels Destillation das chirale Produkt R- bzw. S-2-Butanol aus dem 2-Butanol/2-Butanon-Gemisch in einer chemischen Reinheit > 99 % und mit einem Enantiomerenüberschuss > 98 % gewonnen werden.

Die einzusetzende Konzentration am Substrat 2-Butanon liegt im erfindungsgemäßen Verfahren bevorzugt über 5 Vol.-%, besonders bevorzugt im Bereich zwischen 10 Vol.-% bis 25 Vol.-%.

Die Konzentration des Co-Enzyms NAD(P)H bezogen auf die wässrige Phase beträgt von 0,01 mM bis 10 mM, insbesondere von 0,1 mM bis 1 mM.

Der im Verfahren eingesetzten wässerigen Phase wird bevorzugt ein Puffer zugesetzt, beispielsweise Kaliumphosphat-, Tris/HCl- oder Triethanolamin-Puffer mit einem pH-Wert von 5 bis 10, vorzugsweise ein pH-Wert von 6 bis 9.

Die Carbonylreduktase kann in dem erfindungsgemäßen Verfahren entweder vollständig gereinigt oder teilweise gereinigt, in Form von Zellysaten oder in Form ganzer Zellen eingesetzt werden. Die eingesetzten Zellen können dabei nativ oder permeabilisiert vorliegen.

Die Temperatur beträgt beispielsweise von etwa 10 °C bis 60 °C, bevorzugt von 25 °C bis 35 °C.

Das erfindungsgemäße Verfahren kann beispielsweise in einem geschlossen Reaktionsgefäß aus Glas oder Metall durchgeführt werden. Dazu werden die Komponenten einzeln in das Reaktionsgefäß überführt und unter einer Atmosphäre von beispielsweise Stickstoff oder Luft gerührt. Die Reaktionszeit beträgt von 1 Stunde bis 48 Stunden, insbesondere von 2 Stunden bis 24 Stunden.

Anstelle der Carbonylreduktase aus Candida parapsilosis können auch andere Carbonylreduktasen eingesetzt werden, die in der Lage sind, 2-Butanon enantioseletiv zu S-2-Butanol oder R-2-Butanol zu reduzieren.

Mit den nachfolgenden Beispielen wird die Erfindung noch näher beschrieben.

### Beispiel 1

In diesem Beispiel wird die Herstellung von S-2-Butanol aus 2-Butanon und die Abhängigkeit der Ausbeute an S-2-Butanol in Abhängigkeit vom eingesetzten Verhältnis 2-Butanon/sekundärem Alkohol (2-Heptanol) gezeigt. Als Carbonylreduktase wurde jene aus Candida parapsilosis verwendet. Die Co-Enzymregenerierung erfolgte mit 2-Heptanol. In der nachfolgenden Tabelle 1 sind die Umsatzdaten für drei Ansätze mit unterschiedlichem Verhältnis 2-Butanon/2-Heptanol gezeigt.

**Tabelle 1**

| Zusammensetzung | Ansatz 1 | Ansatz 2 | Ansatz 3 |
|---|---|---|---|
| Puffer (100mM TEA pH = 7.0) | 1 ml | 1 ml | 1 ml |
| 2-Butanon | 2,5 ml | 2,5 ml | 2,5 ml |
| | (0,027 mol) | (0,027 mol) | (0,027 mol) |
| NAD | 0,5 mg | 0,5 mg | 0,5 mg |
| 2-Heptanol | 10 ml | 15 ml | 20 ml |
| | (0,068 mol) | (0,103 mol) | (137 mol) |
| ADH aus Candida parapsilosis | 60 Units | 60 Units | 60 Units |

| Parameter | | | |
|---|---|---|---|
| Volumen | 13,5 ml | 18,5 ml | 23,5 ml |
| Molares Verhältnis | 1: 2,5 | 1: 3,8 | 1: 5 |
| 2-Butanon/ 2-Heptanol | | | |
| Konzentration 2-Butanon in % (v/v) | 18,5% | 13,5% | 10,6% |
| Ausbeute (% S-Butanol) | 43% | 61% | 70% |
| Enantiomeren-Überschuss S-Butanol | 99% S | 99% S | 99% S |

Die Umsetzung wurde durchgeführt, indem zuerst der Puffer in das Reaktionsgefäß gegeben wurde, in welchem dann das NAD und das Enzym gelöst wurden. Anschließend wurden das 2-Heptanol und das 2-Butanon ins Reaktionsgefäß gegeben.

Das Reaktionsgemisch wurde dann unter guter Durchmischung bei 30 °C inkubiert. Der Abbruch der Reaktion erfolgte, wenn keine weitere Umsetzung des 2-Butanon mehr beobachet wurde und somit das Reaktionsgleichgewicht erreicht war.

Der Tabelle 1 ist zu entnehmen, dass die Ausbeute an S-2-Butanol mit zunehmender Konzentration an 2-Heptanol beträchtlich ansteigt.

### Beispiel 2

Mit diesem Beispiel wird an Hand von drei Ansätzen gezeigt, dass die wässerige Phase reduziert werden kann, ohne daß die Ausbeute wesentlich verändert wird.

**Tabelle 2**

| Zusammensetzung | Ansatz 4 | Ansatz 5 | Ansatz 6 |
|---|---|---|---|
| Puffer (100mM TEA pH = 7.0) | 2,5 ml | 5 ml | 10 ml |
| 2-Butanon | 5 ml | 5 ml | 5 ml |
| | (0,054 mol) | (0,054 mol) | (0,054 mol) |
| NAD | 1 mg | 1 mg | 1 mg |
| 2-Heptanol | 30 ml | 30 ml | 30 ml |
| | (0,206 mol) | (0,206 mol) | (0,206 mol) |
| ADH aus Candida parapsilosis | 500 Units | 500 Units | 500 Units |

| Parameter | | | |
|---|---|---|---|
| Volumen | 37,5 ml | 40 ml | 45 ml |
| Molares Verhältnis | 1: 3,8 | 1: 3,8 | 1:3,8 |
| 2-Butanon/ 2-Heptanol | | | |
| Konz. 2-Butanon in % (v/v) | 13,3 % | 12,5 % | 11 % |
| Ausbeute (% S-Butanol) | 55% | 58 % | 5.6 % |
| Enantiomeren-Überschuss S-Butanol | 98 % S | 98 % S | 98 % S |

### Beispiel 3

Mit diesem Beispiel wird gezeigt, dass die Regenerierung des Co-Enzyms mit verschiedenen sekundären Alkoholen vorgenommen werden kann. Die Ergebnisse von drei Ansätzen ist in der nachfolgenden Tabelle 3 angegeben.

**Tabelle 3**

| Zusammensetzung | Ansatz 7 | Ansatz 8 | Ansatz 9 |
|---|---|---|---|
| Puffer (100mM TEA pH = 7.0) | 20 ml | 20 ml | 6 ml |
| 2-Butanon | 5 ml (0,054 mol) | 5 ml (0,054 mol) | 1 ml (0,0108 mol) |
| NAD | 0,5 mg | 0,5 mg | 1 mg |
| Sekundärer Alkohol | 30 ml | 30 ml | 4 ml |
| | 4-Methyl-2-pentanol | 2-Hexanol | 2-Pentanol |
| | (0,23 mol) | (0,23 mol) | (0,036 mol) |
| ADH aus Candida parapsilosis | 500 Units | 500 Units | 100 Units |

| Parameter | | | |
|---|---|---|---|
| Volumen | 55 ml | 55 ml | 11 ml |
| Molares Verhältnis | 1: 4,3 | 1: 4,3 | 1:3,3 |
| 2-Butanon/ 2-Heptanol | | | |
| Konz. 2-Butanon in % (v/v) | 9% | 9% | 9% |
| Ausbeute (% S-Butanol) | 78% | 70% | 68% |
| Enantiomeren-Überschuss S-Butanol | 96% S | 98% S | 98% S |

### Beispiel 4

Mit diesem Beispiel wird die präparative Herstellung von S-2-Butanol im technischen Maßstab gezeigt.

Zur präparativen Herstellung von S-2-Butanol wurden 4,96 l Puffer (TEA 100 mM, pH = 7.0) in einen auf 30 °C temperierten Rührreaktor gegeben. Anschließend wurden 4,96 g NAD im Puffer gelöst und 300 000 Units der Carbonylreduktase aus Candida parapsilosis zum Puffer gegeben. Das Reaktionsgemisch wurde mit 76,11 l (60,9 kg) 2-Heptanol überschichtet und anschließend das Substrat 2-Butanon 12,5 l (10 kg) zugegeben.

Im Anschluss wurde die Rührung angeschaltet und das Reaktionsgemisch unter guter Durchmischung 12h inkubiert. Nach 12 h war das 2-Butanon zu 68 % zu S-2-Butanol mit einem Enantiomerenüberschuss von 98,4 % umgesetzt.

Nach Beendigung der Reaktion erfolgte die Abtrennung und Trocknung der 2-Butanon/S-2-Butanol haltigen Heptanolphase. Das 2-Butanon/S-2-Butanol-Gemisch wurd zuerst mittels Destillation aus der Heptanolphase (Siedepunkt ca. 158-161 °C) gewonnen, bevor in einer zweiten Destillation die Trennung von 2-Butanon (Siedepunkt 80 °C) und S-2-Butanol (Siedepunk = 97-100°C) erfolgte.

Auf diese Weise konnte S-2-Butanol in einer chemischen Reinheit > 99 % erhalten werden.

## Patentansprüche

1. Verfahren zur Herstellung von 2-Butanol durch enzymatisch-katalysierte Reduktion von 2-Butanon mit einer Carbonylreduktase und einem Co-Enzym,
**dadurch gekennzeichnet, daß**
(a) eine wässerige Phase, welche die Carbonylreduktase und das Co-Enzym enthält, mit einer alkoholischen Phase, die mit der wässerigen Phase nicht mischbar ist und 2-Butanon enthält, zur Reduktion des 2-Butanons in Kontakt gebracht wird, mit der Maßgabe, daß der in der alkoholischen Phase vorhandene Alkohol ein sekundärer Alkohol ist, der in der Lage ist, das Co-Enzym zu regenerieren und einen Siedepunkt besitzt, der über dem von Wasser liegt, worauf
(b) das gebildete 2-Butanol abgetrennt wird.

2. Verfahren nach Anspruch 1, **dadurch gekennzeichnet, daß** der Alkohol der alkoholischen Phase 2-Pentanol, 2-Hexanol, 2-Heptanol, 2-Oktanol oder 4-Methyl-2-pentanol ist.

3. Verfahren nach Anspruch 2, **dadurch gekennzeichnet, daß** der Alkohol der alkoholischen Phase 2-Heptanol oder 2-Oktanol ist.

4. Verfahren nach einem der Ansprüche 1 bis 3, **dadurch gekennzeichnet, dass** der sekundäre Alkohol der alkoholischen Phase und das 2-Butanon in einem molaren Verhältnis im Bereich zwischen 1:2 bis 1:10 (2-Butanon : sekundärer Alkohol) eingesetzt werden.

5. Verfahren nach Anspruch 4, **dadurch gekennzeichnet, dass** das molare Verhältnis im Bereich zwischen 1:2,5 bis 1:5 liegt.

6. Verfahren nach einem der Ansprüche 1 bis 5, **dadurch gekennzeichnet, dass** das 2-Butanon in einer Menge eingesetzt wird, die mindestens 5 Vol.%, bevorzugt im Bereich von 10 - 25 Vol.-%, bezogen auf das gesamte Reaktionsgemisch, ist.

7. Verfahren nach einem der Ansprüche 1 bis 6, **dadurch gekennzeichnet, dass** mindestens 2.000 Einheiten, bevorzugt aber mindestens 10.000 Einheiten Carbonylreduktase pro kg 2-Butanon eingesetzt werden.

8. Verfahren nach einem der Ansprüche 1 bis 7, **dadurch gekennzeichnet, dass** eine Carbonylreduktase eingesetzt wird, die von Candida parapsilosis erhältlich ist.

9. Verfahren nach einem der Ansprüche 1 bis 8, **dadurch gekennzeichnet, dass** die Abtrennung des gebildeten 2-Butanols durch Destillation erfolgt.

10. Verfahren nach einem der Ansprüche 1 bis 9, **dadurch gekennzeichnet, dass** eine Carbonylreduktase eingesetzt wird, welche die Herstellung von im wesentlichen enantiomer reinem S-2-Butanol oder R-2-Butanol gestattet.

## Claims

1. A process for the preparation of 2-butanol by enzymatic-catalyzed reduction of 2-butanone with a carbonyl reductase and a coenzyme,
**characterized in that**
(a) an aqueous phase, which contains the carbonyl reductase and the coenzyme, is contacted with an alcoholic phase, which is not miscible with the aqueous phase and contains 2-butanone, in order to reduce the 2-butanone, with the proviso that the alcohol present in the alcoholic phase is a secondary alcohol capable of regenerating the coenzyme and exhibiting a boiling point which lies above that of water, whereupon
(b) the 2-butanol formed is separated.

2. A process according to claim 1, **characterized in that** the alcohol of the alcoholic phase is 2-pentanol, 2-hexanol, 2-heptanol, 2-octanol or 4-methyl-2-pentanol.

3. A process according to claim 2, **characterized in that** the alcohol of the alcoholic phase is 2-heptanol or 2-octanol.

4. A process according to any of claims 1 to 3, **characterized in that** the secondary alcohol of the alcoholic phase and the 2-butanone are used at a molar ratio ranging from 1:2 to 1:10 (2-butanone : secondary alcohol).

5. A process according to claim 4, **characterized in that** the molar ratio ranges from 1:2.5 to 1:5.

6. A process according to any of claims 1 to 5, **characterized in that** the 2-butanone is used in an amount of at least 5% by volume, preferably ranging from 10 - 25% by volume, based on the total reaction mixture.

7. A process according to any of claims 1 to 6, **characterized in that** at least 2,000 units, preferably, however, at least 10,000 units, of carbonyl reductase per kg of 2-butanone are used.

8. A process according to any of claims 1 to 7, **characterized in that** a carbonyl reductase obtainable from Candida parapsilosis is used.

9. A process according to any of claims 1 to 8, **characterized in that** the 2-butanol formed is separated by distillation.

10. A process according to any of claims 1 to 9, **characterized in that** a carbonyl reductase is used which permits the preparation of substantially enantiomerically pure S-2-butanol or R-2-butanol.

## Revendications

1. Procédé de préparation du 2-butanol par réduction de la 2-butanone, catalysée par une enzyme, avec une carbonyle réductase et une co-enzyme,
**caractérisé en ce que**
(a) une phase aqueuse qui contient la carbonyle réductase et la co-enzyme, est amenée en contact avec une phase alcoolique qui n'est pas miscible à la phase aqueuse et qui contient la 2-butanone, afin de réduire la 2-butanone, à la condition que l'alcool présent dans la phase alcoolique soit un alcool secondaire qui est en mesure de régénérer la co-enzyme et qui possède un point d'ébullition qui se trouve au-dessus de celui de l'eau, de laquelle
(b) le 2-butanol formé est séparé.

2. Procédé selon la revendication 1, **caractérisé en ce que** l'alcool de la phase alcoolique est le 2-pentanol, le 2-hexanol, le 2-heptanol, le 2-octanol ou le 4-méthyl-2-pentanol.

3. Procédé selon la revendication 2, **caractérisé en ce que** l'alcool de la phase alcoolique est le 2-heptanol ou le 2-octanol.

4. Procédé selon l'une quelconque des revendications 1 à 3, **caractérisé en ce que** l'alcool secondaire de la phase alcoolique et la 2-butanone sont mis en oeuvre dans un rapport molaire dans la plage comprise entre 1:2 à 1:10 (2-butanone:alcool secondaire).

5. Procédé selon la revendication 4, **caractérisé en ce que** le rapport molaire se trouve dans la plage comprise entre 1:2,5 à 1:5.

6. Procédé selon l'une quelconque des revendications 1 à 5, **caractérisé en ce que** la 2-butanone est mise en ouvre en une quantité qui est au moins de 5% en volume, de préférence dans la plage de 10 à 25% en volume, par rapport au mélange réactionnel total.

7. Procédé selon l'une quelconque des revendications 1 à 6, **caractérisé en ce qu'**au moins 2 000 unités, de préférence toutefois au moins 10 000 unités de carbonyle réductase sont mises en oeuvre par kg de 2-butanone.

8. Procédé selon l'une quelconque des revendications 1 à 7, **caractérisé en ce qu'**une carbonyle réductase est mise en oeuvre, que l'on peut obtenir à partir de *Candida parapsilosis.*

9. Procédé selon l'une quelconque des revendications 1 à 8, **caractérisé en ce que** la séparation du 2-butanol formé se réalise par distillation

10. Procédé selon l'une quelconque des revendications 1 à 9, **caractérisé en ce qu'**une carbony7.e réductase est mise en oeuvre, laquelle permet la préparation de S-2-butanol ou de R-2-butanol, énantiomères essentiellement purs.
